# EUROPEAN PATENT SPECIFICATION

(11) **EP 1 041 973 B1**
(45) Date of publication and mention of the grant of the patent: **23.11.2005**
(21) Application number: 98966980.9
(22) Date of filing: 29.12.1998
(51) Int. Cl.: A61K 9/50

(54) **MICROCAPSULES; PROCESS FOR PREPARING MICROCAPSULES CONTAINING A BIOLOGICALLY ACTIVE COMPOUND AND LIQUID SOLUTION**
MIKROKAPSELN UND VERFAHREN ZUR HERSTELLUNG VON MIKOROKAPSELN ENTHALTEND EIN BIOLOGISCH AKTIV WIRKSTOFF UND EINE LÖSUNG
MICROCAPSULES, PROCEDE DE PREPARATION DE MICROCAPSULES CONTENANT UN COMPOSE BIOLOGIQUEMENT ACTIF ET SOLUTION LIQUIDE

(30) Priority: 30.12.1997 BR 9706494
(43) Date of publication of application: 11.10.2000
(73) Proprietor: Natura Cosméticos S.A., Sao Paolo, Sao Paolo (BR)
(72) Inventor: POMMEZ, Philippe, Joseph, Sao Paulo, SP (BR); ALC NTARA MARTINS ZUCCHETTI, Roberto, Sao Paulo, SP (BR)
(74) Representative: Senior, Janet
(86) International application number: PCT/BR1998/000111
(87) International publication number: WO 1999/036059

(56) References cited:
- US-A- 4 518 547
- US-A- 5 395 620
- PATENT ABSTRACTS OF JAPAN vol. 016, no. 207 (C-0941), 18 May 1992 (1992-05-18) & JP 04 036233 A (BIOMATERIAL UNIVERSE), 6 February 1992 (1992-02-06)
- PATENT ABSTRACTS OF JAPAN vol. 013, no. 236 (C-602), 30 May 1989 (1989-05-30) & JP 01 043343 A (POLA CHEM. IND. INC.), 15 February 1989 (1989-02-15)
- DATABASE WPI Week 8735 Derwent Publications Ltd., London, GB; AN 87-246478 [35] XP002110493 & JP 62 168539 A (FUJI PHOTO FILM CO. LTD.) 24 July 1987 (1987-07-24)

## Description

### Field of the Invention:

This invention refers to microcapsules containing biologically active compounds, destined to make available said active compounds inside an individual, such as in certain skin layers, digestive tract or blood circulation.

The invention also discloses a process for the preparation of microcapsules containing a biologically active compound and a liquid solution having a viscous flow of a pre-established degree.

### Of the prior art:

As it is well known in the art, microcapsules are capsules of microscopical size, adapted for containing and dispensing, in an advantageous manner, a biologically active compound, such as a cosmetic, pharmaceutical or nutritional compound, to be administered to an individual in need of such ingredients to prevent or solve some organic problem. Said microcapsules are discussed, for instance, in US Patent 5.395.620 (Huc et al) the disclosures of which are hereby incorporated by reference.

Said microcapsules, preferably made of organic material, have the function of conveying and dispensing the biologically active compound contained within them, to a certain site or region of the individual, preserving and maintaining the proprieties of said compound.

Said microcapsules are particularly adapted for the administration of vitamins to individuals. When referring to C vitamin used topically with the purpose of stimulating the synthesis of collagen for the correction of skin imperfections as wrinkles and others, it is desirable that the biologically active compound, C vitamin in this case, be released below the corneous layer of the skin. Thus, microcapsules made of organic materials are particularly useful for this type of application, since they are compatible with the live beings organisms, and are easily degradable without impairing individual or environment.

However, a common problem to all microcapsules is the difficulty of keeping the biologically active compounds in their interior, since the latter are usually made of materials that permeate the reticules of the microcapsules, and are released before reaching the selected site in the individual.

So far, the problem of keeping the biologically active compounds within the microcapsules has been mainly solved by the modification of the structure, and specially of the walls of the microcapsule, with the purpose of rendering it impermeable to its contents, and particularly to the biologically active compound.

In spite of such approach actually solving the problem of the undesired outflow of the biologically active compound, it also presents, among others, the inconvenience of causing the loss of part of the biophysical-chemical properties of the capsules, due to the changes in its structure. Examples of this and other approaches, including the use of liposome capsules, the disadvantage of which is the difficult selection of ingredients for their composition, are disclosed in Patents JP 8325117, US 5.069.936, JP 2293041, JP 2164439, EP 0 207 655, DE 2706705, BE 873865, US 5.160.529, US 5.332.584, US 4.789.516, US 4.532.123, US 5.656.469 e US 5.453.368, among others.

Considering that such containers may present a similar degree of reticulation, another disadvantage of modifying the microcapsules is the need to develop a specific and compatible container for each biologically active compound to be used, thus rendering the process of manufacturing microcapsules containing biologically active compounds a very expensive one.

### Scope of the invention

The major purpose of this invention is to minimize the undesired outflow of a biologically active compound from a microcapsule, without modifying the structure of the latter, so that the ability of releasing the contents of the microcapsule may occur in a retarded manner, or even a programmed one. Which is to say, so that the release of the biologically active compounds is initiated as of a certain moment, in a certain region of the individual, after its introduction in the organism of the individual.

In this sense, and contrary to prior techniques, this invention has as its premise the modification of the properties of the biologically active compound itself, so that it will present a reduced ability to outflow, keeping unaltered the microcapsule that contains it.

Thus, a first aspect of the invention foresees the association of the biologically active compound to a liquid solution capable of reducing its outflow. In a preferential embodiment, it is foreseen the association of the biologically active compound to be contained in the microcapsule to a solution of viscous flow. Such an association may occur through a substantial dissolution of said compound in the liquid solution, prior to its introduction in the container.

Viscous flow, unless otherwise specified, is hereby defined as any flow of a liquid where viscosity is different from zero. Therefore, any biologically active compound associated to a solution with a viscous flow lies within the scope of the invention.

In view of the above considerations, the purpose of this invention is reached by means of a microcapsule characterized by the fact of being the carrier of a biologically active compound associated to a liquid solution capable of reducing the outflow of said compound from said container.

One other aspect of this invention refers to a process for preparing microcapsules containing a biologically active compound, characterized by the fact of comprising a step of associating said compound to a liquid solution capable of reducing the outflow of said compound from said container.

This invention refers yet to a liquid solution containing a biologically active compound, adapted to be inserted into a microcapsule, and characterized by the fact of presenting a viscous flow of a predetermined level.

### Detailed Description of the Invention:

As already mentioned above, the basic premise of this invention is the association of a biologically active compound to be contained in a microcapsule, to a liquid solution capable of reducing the outflow of said biologically active compound from its microcapsule.

According to a preferable embodiment which is solely given as an example, said liquid solution presents a viscous flow of a predetermined degree, to prevent the outflowing of the compound through the reticules of the container at an undesired occasion, such as, for instance, before reaching a certain region within the individual who received it.

The solution, in which said compound was dissolved before its introduction in the container, may be alcohol or aqueous, depending in the solubility of the biologically active compound, and shall contain at least one polymer dissolved therein, such as polyvynilalcohol or polyvynilthioalcohol and their combinations.

Studies performed by the Applicant have shown that the degree of viscosity of said liquid solution can be advantageously determined by the variation of the concentration of said polymer in a substantially reverse ratio to its molecular weight. Since the aqueous solution given hereby as an example should present a viscosity greater than about 4 mPa.s., and preferably greater than about 8 mPa.s., the molecular weight of said polymers may be in the range of 70,000 g/mol to 200,000 g/mol, while the concentration of said polymers may be of about 1% w/v to about 5% w/v, with a greater preference for about 1% w/v.

The organic microcapsules are advantageously prepared from a matrix containing selected molecules from the group comprising protein molecules, glycosamineglicanes molecules, polysaccharides molecules, and the combinations thereof. The protein and glycosamineglicanes molecules may be derived from the sea, or from terrestrial mammals, while the molecules of the useful polysaccharides are of vegetable origin. In a preferred way, the protein molecules are collagen molecules, and the glycosamineglicanes molecules are chondroitin sulfate molecules. The polysaccharide molecules are, preferably, galactomannose molecules.

The preferred organic microcapsules have a substantianlly globular shape, and present a statistical distribution of size smaller than 100 micra, and , more preferably, between 3 and 60 micra.

The biologically active compound to be introduced in the organic containers is selected among cosmetic, pharmaceutical or nutritional compounds, such as for instance the various compounds identified as C Vitamin, which include but are not limited to levorotatory ascorbic acid, its salts, esters, derivations and combinations.

One must bear in mind that the above description refers only to one exemplificating embodiment of this invention, the scope of which is defined in the following Claims:

## Claims

1. A liquid solution adapted to be introduced into a microcapsule and comprising a biologically active compound and at least one polymer that is present in a concentration of 1% to 5% by weight based on the total volume of the solution, wherein the solution has a viscosity greater than 4 mPa.s.

2. A liquid solution according to claim 1, **characterized in that** the viscosity of said liquid solution is greater than 8 mPa.s.

3. A liquid solution according to claims 1 or 2, **characterized in that** said polymer has a molecular weight ranging from 70,000 g/mol to 200,000 g/mol.

4. A liquid solution according to any of the preceding claims, **characterized in that** the polymer is present in a concentration of about 1% weight/volume.

5. A liquid solution according to any of the preceding claims, **characterized in that** said polymer is selected from the group comprising polyvinylalcohol, polyvinylthioalcohol and mixtures thereof.

6. A liquid solution according to any of the preceding claims, **characterized in that** said polymer is substantially dissolved in the liquid solution.

7. A liquid solution according to any of the preceding claims, **characterized in that** said microcapsule is made of organic material.

8. A liquid solution according to claim 7, **characterized in that** the said organic material is selected from groups of molecules derived from the sea, or from terrestrial mammals, or of vegetable origin, and combinations thereof.

9. A liquid solution according to claim 8, **characterized in that** the organic material is selected from glycoseamineglicanes, proteins, polysaccharides and combinations thereof.

10. A liquid solution according to claim 9, **characterized in that** the protein is collagen, the glycoamineglicane is chondroitin sulfate, and the polysaccharide is galactomannose.

11. A liquid solution according to any of the claims 7 to 9, **characterized in that** the organic material is selected from collagen, atelocollagen, polyholosides, chondroitin sulfate, their byproducts and their combinations.

12. A liquid solution according to any of the daims 1 to 11, **characterized in that** the biologically active compound is selected from cosmetic, pharmaceutical and nutritional compounds.

13. A liquid solution according to claim 12, **characterized in that** the biologically active compound is a vitamin.

14. A liquid solution according to claim 13, **characterized in that** the vitamin is selected from levorotatory ascorbic acid, its salts, esters and derivatives and combinations thereof.

15. A microcapsule comprising the liquid solution as defined in any of claims 1 to 14.

16. A microcapsule for treatment of the human body for cosmetic, pharmaceutical or nutritional purposes, comprising the liquid solution as defined in any of claims 1 to 14.

17. Process for the production of the liquid solution of claim 1, comprising dissolving a biologically active compound in liquid solution comprising at least one polymer that is present in a concentration of 1% to 5% by weight based on the total volume of the solution and wherein the solution has a viscosity greater than 4mPa.s.

18. A process as claimed in claim 17, wherein the liquid solution is as specified in any one of claims 2 to 14.

19. A process for the production of a microcapsule containing a biologically active compound, comprising preparing a liquid solution according to claim 17 or claim 18 and introducing said solution into the microcapsule.

20. Use of a liquid solution as defined in any of claims 1 to 14, for the manufacture of a microcapsule for treatment of the human body for cosmetic, pharmaceutical or nutritional purposes.

21. Use of a polymer for the manufacture of a microcapsule as defined in claim 15 to minimise the outflow of the biologically active material from the microcapsule before it reaches the selected site in an individual.

22. A method for the cosmetic treatment of the human body, which comprises contacting the skin with a microcapsule as defined in claim 15.

## Patentansprüche

1. Flüssige Lösung, die ausgebildet ist, um in eine Mikrokapsel eingebracht zu werden, und eine biologisch aktive Verbindung und zumindest ein Polymer, das in einer Konzentration von 1 bis 5 Gew.-% basierend auf dem Gesamtvolumen der Lösung vorhanden ist, aufweist, worin die Lösung eines Viskosität von mehr als 4 mPa·s hat.

2. Flüssige Lösung nach Anspruch 1, **dadurch gekennzeichnet, dass** die Viskosität der flüssigen Lösung größer als 8 mPa·s ist.

3. Flüssige Lösung nach Anspruch 1 oder 2, **dadurch gekennzeichnet, dass** das Polymer ein Molekulargewicht im Bereich von 70000 g/mol bis 200000 g/mol hat.

4. Flüssige'Lösung nach einem der vorhergehenden Ansprüche, **dadurch gekennzeichnet, dass** das Polymer in einer Konzentration von etwa 1% Gewicht/Volumen vorhanden ist.

5. Flüssige Lösung nach einem der vorhergehenden Ansprüche, **dadurch gekennzeichnet, dass** das Polymer ausgewählt ist aus der Gruppe aufweisend Polyvinylalkohol, Polyvinylthioalkohol und Mischungen hiervon.

6. Flüssige Lösung nach einem der vorhergehenden Ansprüche, **dadurch gekennzeichnet, dass** das Polymer im Wesentlichen in der flüssigen Lösung aufgelöst ist.

7. Flüssige Lösung nach einem der vorhergehenden Ansprüche, **dadurch gekennzeichnet, dass** die Mikrokapsel aus organischem Material besteht.

8. Flüssige Lösung nach Anspruch 7, **dadurch gekennzeichnet, dass** das organische Material ausgewählt ist aus Gruppen von Molekülen, die aus der See oder von Landsäugetieren gewonnen oder pflanzlichen Ursprungs sind, sowie Kombinationen hiervon.

9. Flüssige Lösung nach Anspruch 8, **dadurch gekennzeichnet, dass** das organische Material ausgewählt ist aus Glukoseaminglykanen, Proteinen, Polysacchariden und Kombinationen hiervon.

10. Flüssige Lösung nach Anspruch 9, **dadurch gekennzeichnet, dass** das Protein Kollagen ist, die Glykoaminglikane Chondroitinsulfat sind und die Polysaccharide Galactomannose sind.

11. Flüssige Lösung nach einem der Ansprüche 7 bis 9, **dadurch gekennzeichnet, dass** das organische Material ausgewählt ist aus Kollagen, Atelokollagen, Polyholosiden, Chondroitinsulfat, ihren Nebenprodukten und ihren Kombinationen.

12. Flüssige Lösung nach einem der Ansprüche 1 bis 11, **dadurch gekennzeichnet, dass** die biologisch aktive Komponente ausgewählt ist aus kosmetischen, pharmazeutischen und Nahrungsmittelverbindungen.

13. Flüssige Lösung nach Anspruch 12, **dadurch gekennzeichnet, dass** die biologisch aktive Verbindungen Vitamin ist.

14. Flüssige Lösung nach Anspruch 13, **dadurch gekennzeichnet, dass** das Vitamin ausgewählt ist aus linksdrehender Ascorbinsäure, ihren Salzen, Estern und Derivaten sowie Kombinationen hiervon.

15. Mikrokapsel, aufweisend die flüssige Lösung nach einem der Ansprüche 1 bis 14.

16. Mikrokapsel für die Behandlung des menschlichen Körpers für kosmetische, pharmazeutische oder Ernährungszwecke, aufweisend die flüssige Lösung nach einem der Ansprüche 1 bis 14.

17. Verfahren für die Herstellung der flüssigen Lösung nach Anspruch 1, aufweisend das Auflösen einer biologisch aktiven Komponente in flüssiger Lösung, die zumindest ein Polymer, das in einer Konzentration von 1 bis 5 Gew.-% basierend auf dem Gesamtvolumen der Lösung vorhanden ist, aufweist, und wobei die Lösung eine Viskosität von mehr als 4 mPa·s hat.

18. Verfahren nach Anspruch 17, bei dem die flüssige Lösung wie in einem der Ansprüche 1 bis 14 spezifiziert ist.

19. Verfahren zur Herstellung einer Mikrokapsel enthaltend eine biologisch aktive Verbindung, aufweisend die Herstellung einer flüssigen Lösung nach Anspruch 17 oder Anspruch 18 und das Einbringen dieser Lösung in die Mikrokapsel.

20. Verwendung einer flüssigen Lösung nach einem der Ansprüche 1 bis 14 für die Herstellung einer Mikrokapsel zur Behandlung des menschlichen Körpers für kosmetische, pharmazeutische oder Ernährungszwecke.

21. Verwendung eines Polymers für die Herstellung einer Mikrokapsel nach Anspruch 15, um den Austritt des biologisch aktiven Materials aus der Mikrokapsel zu minimieren, bevor sie die ausgewählte Stelle in einem Individuum erreicht.

22. Verfahren für die kosmetische Behandlung des menschlichen Körpers, das den Kontakt der Haut mit einer Mikrokapsel nach Anspruch 15 aufweist.

## Revendications

1. Solution liquide apte à être introduite dans une microcapsule et comprenant un composé actif biologiquement et au moins un polymère qui est présent dans une concentration de 1 à 5 % en poids, rapportée au volume total de la solution, dans laquelle la solution présente une viscosité supérieure à 4 mPa.s.

2. Solution liquide selon la revendication 1, **caractérisée en ce que** la viscosité de ladite solution liquide est supérieure à 8 mPa.s.

3. Solution liquide selon les revendications 1 ou 2, **caractérisée en ce que** ledit polymère présente un poids moléculaire allant de 70 000 g/mole à 200 000 g/mole.

4. Solution liquide selon l'une quelconque des revendications précédentes, **caractérisée en ce que** le polymère est présent dans une concentration d'environ 1 % poids/volume.

5. Solution liquide selon l'une quelconque des revendications précédentes, **caractérisée en ce que** ledit polymère est choisi dans le groupe comprenant de l'alcool polyvinylique, de l'alcool thiopolyvinylique et leurs mélanges.

6. Solution liquide selon l'une quelconque des revendications précédentes, **caractérisée en ce que** ledit polymère est sensiblement dissous dans la solution liquide.

7. Solution liquide selon l'une quelconque des revendications précédentes, **caractérisée en ce que** ladite microcapsule est à base de matière organique.

8. Solution liquide selon la revendication 7, **caractérisée en ce que** ladite matière organique est choisie dans les groupes de molécules dérivées de la mer, ou de mammifères terrestres, ou d'origine végétale, et leurs combinaisons.

9. Solution liquide selon la revendication 8, **caractérisée en ce que** la matière organique est choisie à partir de glycosaminoglycannes, protéines, polysaccharides et leurs combinaisons.

10. Solution liquide selon la revendication 9, **caractérisée en ce que** la protéine est le collagène, le glycosaminoglycannes est le sulfate de chondroïtine, et le polysaccharide est la galactomannose.

11. Solution liquide selon l'une quelconque des revendications 7 à 9, **caractérisée en ce que** la matière organique est choisie à partir de collagène, atélocollagène, polyholosides, sulfate de chondroitine, leurs sous-produits et leurs combinaisons.

12. Solution liquide selon l'une quelconque des revendications 1 à 11, **caractérisée en ce que** le composé actif biologiquement est choisi à partir de composés cosmétiques, pharmaceutiques et nutritionnels.

13. Solution liquide selon la revendication 12, **caractérisée en ce que** le composé actif biologiquement est une vitamine.

14. Solution liquide selon la revendication 13, **caractérisée en ce que** la vitamine est choisie à partir d'acide ascorbique lévorotatif, ses sels, esters et dérivés et leurs combinaisons.

15. Microcapsule comprenant la solution liquide telle que définie dans l'une quelconque des revendications 1 à 14.

16. Microcapsule pour le traitement du corps humain à des fins cosmétiques, pharmaceutiques ou nutritionnelles, comprenant la solution liquide telle que définie dans l'une quelconque des revendications 1 à 14.

17. Procédé pour la production de la solution liquide de la revendication 1, comprenant la dissolution d'un composé actif biologiquement dans la solution liquide comprenant au moins un polymère qui est présent dans une concentration de 1 à 5 % en poids, rapportée au volume total de la solution, et dans lequel la solution a une viscosité supérieure à 4 mPa.s.

18. Procédé selon la revendication 17, dans lequel la solution liquide est telle que spécifiée dans l'une quelconque des revendications 2 à 14.

19. Procédé pour la production d'une microcapsule contenant un composé actif biologiquement, comprenant la préparation d'une solution liquide selon la revendication 17 ou la revendication 18 et l'introduction de ladite solution dans la microcapsule.

20. Utilisation d'une solution liquide telle que définie dans l'une quelconque des revendications 1 à 14 pour la fabrication d'une microcapsule pour le traitement du corps humain à des fins cosmétiques, pharmaceutiques ou nutritionnelles.

21. Utilisation d'un polymère pour la fabrication d'une microcapsule telle que définie dans la revendication 15 pour réduire l'écoulement vers l'extérieur de la matière active biologiquement de la microcapsule avant qu'elle n'atteigne le site choisi chez un individu.

22. Procédé pour le traitement cosmétique du corps humain qui comprend la mise en contact de la peau avec une microcapsule telle que définie dans la revendication 15.
